# EUROPEAN PATENT SPECIFICATION

(11) **EP 2 117 469 B1**
(45) Date of publication and mention of the grant of the patent: **09.07.2014**
(21) Application number: 08725248.2
(22) Date of filing: 05.02.2008
(51) Int. Cl.: A61F 2/24

(54) **Percutaneous valve system**
Perkutanes Klappensystem
Système de valve percutanée

(30) Priority: 05.02.2007 US 899444 P
(43) Date of publication of application: 18.11.2009
(73) Proprietor: Boston Scientific Limited, Christ Church (BB)
(72) Inventor: HILL, Jason, P., Brooklyn Park, MN 55443 (US); EIDENSCHINK, Tracee, E.J., Wayzata, MN 55391 (US); KVEEN, Graig, L., Maple Grove, MN 55311 (US)
(74) Representative: Schley, Jan Malte
(86) International application number: PCT/US2008/001591
(87) International publication number: WO 2008/097590

(56) References cited:
- EP-A- 1 723 935
- US-A1- 2003 040 792
- US-A1- 2006 259 137
- US-B1- 6 315 792

## Description

The present disclosure relates generally to systems for use in the vascular system; and more particularly to a valve system for use in the vasculature system.

Valves can become damaged and/or diseased for a variety of reasons. Damaged and/or diseased valves are grouped according to which valve or valves are involved, and the amount of blood flow that is disrupted by the damaged and/or diseased valve. The most common valve diseases occur in the mitral and aortic valves. Diseases of the tricuspid and pulmonary valves are fairly rare.

The aortic valve regulates the blood flow from the heart's left ventricle into the aorta. The aorta is the main artery that supplies oxygenated blood to the body. As a result, diseases of the aortic valve can have a significant impact on an individual's health. Examples of such diseases include aortic regurgitation and aortic stenosis.

Aortic regurgitation is also called aortic insufficiency or aortic incompetence. It is a condition in which blood flows backward from a widened or weakened aortic valve into the left ventricle of the heart. In its most serious form, aortic regurgitation is caused by an infection that leaves holes in the valve leaflets. Symptoms of aortic regurgitation may not appear for years. When symptoms do appear, it is because the left ventricle must work harder relative to an uncompromised aortic valve to make up for the backflow of blood. The ventricle eventually gets larger and fluid backs up.

Aortic stenosis is a narrowing or blockage of the aortic valve. Aortic stenosis occurs when the valve leaflets of the aorta become coated with deposits. The deposits change the shape of the leaflets and reduce blood flow through the valve. Again, the left ventricle has to work harder relative to an uncompromised aortic valve to make up for the reduced blood flow. Over time, the extra work can lead to an enlargement of the heart muscle.

EP 1 723 935 A discloses a prosthetic heart valve system including a prosthetic heart valve and a deflection device. The deflection device includes a line and a connector assembly including a tensioning component. The line interconnects and passes through free ends of stent posts associated with the heart valve, and is further connected to the tensioning component. The tensioning component is transitionable to a tensioning state in which the line is tensioned to inwardly deflect the stent posts.

Further devices have been described in US 2006/259137 A1, US 2003/040792 A1 and US 6 315 792 B1.
Figure 1 illustrates an example of a valve according to the present disclosure.
Figure 2 illustrates an example of a valve according to the present disclosure.
Figure 3 illustrates an example of a valve according to the present disclosure.
Figure 4 illustrates an example of a valve according to the present disclosure.
Figures 5A and 5B illustrate a cross-sectional view of an embodiment of a system that includes a valve according to the present disclosure.
Figure 5C illustrates a balloon catheter used with an embodiment of the system that includes a valve according to the present disclosure.

The present invention relates a system according to claim 1.

Embodiments of the present invention are directed to a system for percutaneous cardiac or venous valve replacement and/or augmentation. For example, the system can include a valve that can be used to replace an incompetent valve (e.g., an aortic valve, a mitral valve, a tricuspid valve, a pulmonary valve, or a venous valve) in a body lumen. Embodiments of the valve include a valve frame, a valve leaflet coupled to the valve frame, and a thread passing over the valve frame to hold the valve frame in a partially deployed state, where removal of the thread allows the valve frame to expand toward a deployed state. In one example, embodiments of the present disclosure may help to augment or replace the function of a valve of individuals having heart valve disease or suffering from chronic venous insufficiency.

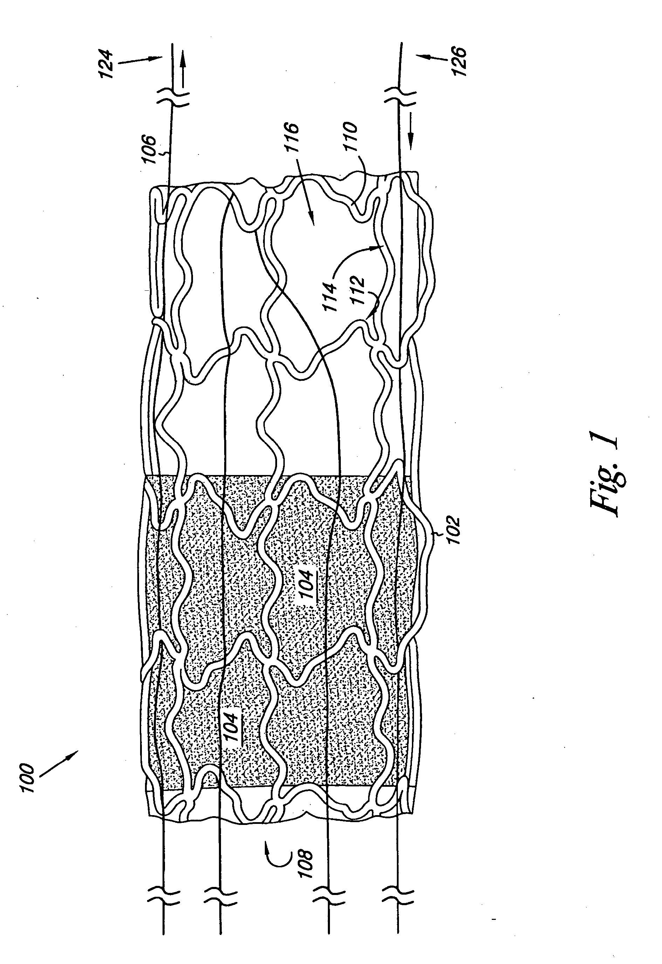

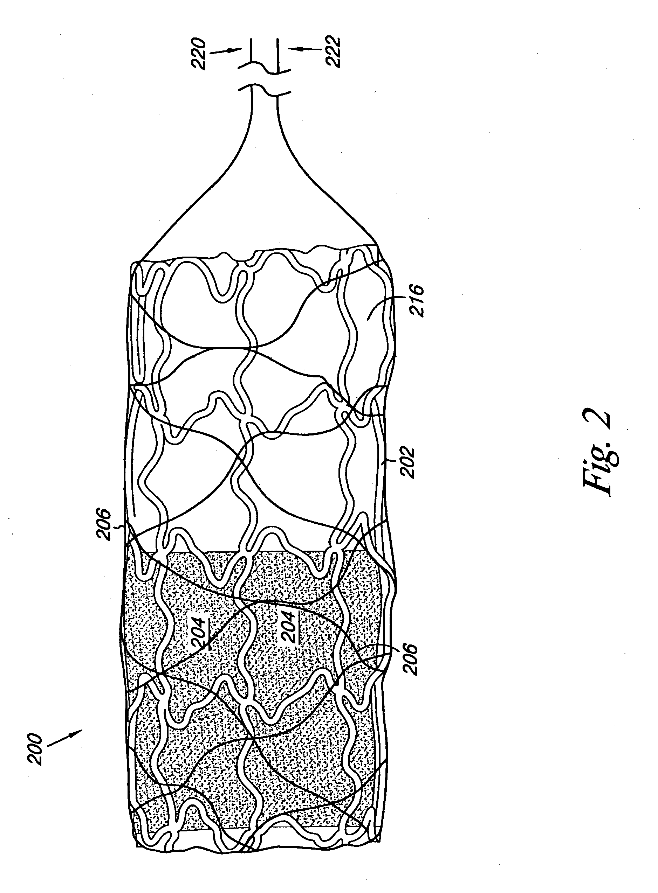

The figures herein follow a numbering convention in which the first digit or digits correspond to the drawing figure number and the remaining digits identify an element or component in the drawing. Similar elements or components between different figures may be identified by the use of similar digits. For example, 110 may reference element "10" in Fig. 1, and a similar element may be referenced as 210 in Fig. 2. As will be appreciated, elements shown in the various embodiments herein can be added, exchanged, and/or eliminated so as to provide any number of additional embodiments of valve and/or system. In addition, as will be appreciated the proportion and the relative scale of the elements provided in the figures are intended to illustrate the embodiments of the present invention, and should not be taken in a limiting sense.

Various embodiments of the present disclosure are illustrated in the figures. Generally, the valve can be implanted within the fluid passageway of a body lumen, such as for replacement or augmentation of a cardiac valve structure or venous valve structure within the body lumen (e.g., aortic and venous valves), to regulate the flow of a bodily fluid through the body lumen in a single direction.

The embodiments of the valve of the present disclosure allow for the valve frame to be held and/or restrained in a partially deployed state. As used herein, a partially deployed state of the valve frame lies between an undeployed state (i.e., the state of the valve frame at the time the valve is outside the body) and a deployed state (i.e., the state of the valve frame at the time the valve is to be left in the body).

In the various embodiments, holding the valve frame in the partially deployed state allows the valve to be better positioned in a desired location prior to its final deployment. In this partially deployed state, the position of the valve relative the desired implant location can be adjusted to correct any foreshortening and/or stent jump that can occur in self-expanding valve frames and some balloon expandable valve frames as they expand from the small compressed undeployed state.

In addition, having the valve in the partially deployed state prior to completing the deployment allows for adjustments of the valve position relative native structures in the region of the implant site (e.g., the coronary ostia). In addition, holding the valve in the partially deployed state allows blood from the still beating heart to perfuse around the partially deployed valve to provide oxygenated blood to the heart and brain. This staged deployment of the valve of the present disclosure is in contrast to valves that are deployed without the advantage of temporarily pausing at an intermediate deployment stage (i.e., the partial deployment state) to allow for adjustments in the placement of valve prior to full deployment.

Figure 1 provides an embodiment of a valve 100 of the present disclosure. The valve 100 includes a valve frame 102, a valve leaflet 104, and a thread 106 passing over the valve frame 102. As illustrated, the thread 106 passing over the valve frame 102 serves to hold the valve frame 102 in a partially deployed state, as discussed herein.

For the various embodiments, the thread 106 can have a number of different configurations. For example, the thread 106 can be a monofilament (i.e., a single strand of material). Alternatively, the thread 106 can have a multistrand configuration. For example, the thread 106 having multiple strands can have a woven, a braided, and/or a twisted configuration. Combinations of these configurations are also possible.

The thread 106 can also have a multilayer construction, where the thread 106 includes a core that is surrounded by one or more layers. The core and layers of the thread 106 can be formed of different materials and/or the same materials having different desired properties. In addition, the thread 106 can further include a coating that does not necessarily constitute a "layer" (i.e., a coating can include a material that imbeds or integrates into the layer on which it is applied). Such layers and/or coatings can impart properties to the thread 106 such as hardness and/or lubricity, among others.

The thread 106 can be formed of a number of materials. Such materials can have a sufficient tensile strength and yield point to resist stretching so as to hold the valve frame 102 in the partially deployed state as discussed herein. Examples of such materials include, but are not limited to, polymers such as nylon(s), acetal, Pebax, PEEK, polyamide, polypyrol, PTFE, e-PTFE, PET and Kevlar. Alternatively, the deployment thread 656 can be formed of metal and/or metal alloys, such as Stainless Steel alloys (304, 316, 17-7 PH, 17-4 PH, etc.), Cobalt Alloys (Elgiloy, L605, MP35, etc.), Nitinol, Nb-1Zr, Tungsten, Molybdenum, and titanium. Other polymers, metals and/or metal alloys are also possible. The thread 106 could also be coated with a lubricious material, such as a hydrophilic coating. The materials of the deployment thread 106 also include combinations of these materials in one or more of the configurations as discussed herein.

As illustrated, the valve frame 102 includes frame members 110 having an inner surface 112 that helps to define the lumen 108, and an outer surface 114 opposite the inner surface 112. The frame members 110 also define cells 116 of the valve frame 102. In one embodiment, the thread 106 passes over a portion of the outer surface 114 and a portion of the inner surface 112 through the openings of the cells 116 to help hold the valve frame 102 in the partially deployed state. The edges of the frame members 110 can be polished and contoured.

In one embodiment, the thread 106 passes over the frame 102 at defined locations and in a predefined pattern that allows the thread 106 to hold the valve 100 in the partially deployed state. Such defined locations on the valve frame 102 can include joints and/or corners of the frame members 110 (e.g., where two or more of the frame members 110 join). In the various embodiments, the thread 106 passing over these structures can carry at least a portion of the tension from the frame members trying to expand to the deployed state. Other structures on the valve frame 102 from which to hold the valve 100 with the thread are also possible (e.g., eyelets, notches, etc.), and will be discussed herein.

As discussed, the thread 106 can pass over the valve frame 102 in a predefined configuration that allows the thread 106 to hold the valve 100 in the partially deployed state. In one embodiment, the thread 106 can extend from the valve frame 102 to be releasably attached to one or more anchors located on an adjacent structure. One example of such a structure is a delivery catheter. The anchors on the adjacent structure can provide one or more locations from which to help restrain or tether the valve frame 102 in its partially deployed state. Embodiments illustrating this aspect of the present disclosure are discussed further herein.

The predefined configurations can include those in which the thread 106 passes over the valve frame 102 in one or both of a longitudinal and/or a radial direction relative the longitudinal axis of the valve frame 102. Examples of such a predefined configuration can further include a woven configuration, a grid configuration, and/or an intertwined configuration. Other configurations for the pattern of the thread 106 relative the valve frame 102 are also possible.

For the various embodiments, the thread 106 can be a single length of thread that extends between a first end 124 and a second end 126. Alternatively, the thread 106 can include one or more branches that extend from a main thread body. In an additional embodiment, the thread 106 can include two or more lengths that are used together in holding the valve frame 102 in the partially deployed state, as discussed herein. When two or more lengths of the thread 106 are used, they can be used separately to contact the valve frame 102, but not each other. Alternatively, two or more lengths of the thread 106 can be intertwined, crossed and/or physically associated with each other so as to hold the valve frame 102 in the partially deployed state.

As discussed, the thread 106 can hold the valve frame 102 in the partially deployed state until it is removed from the valve 100. In one embodiment, the thread 106 holds the valve frame 102 in the partially deployed state that is fifty (50) to ninety-five (95) percent of the deployed state. Other percentages of the deployed state are possible (e.g., eighty (80) to ninety-five (95) percent of the deployed state).

For the various embodiments, the valve frame 102 in the partially deployed state holds the thread 106 under tension. When the thread 106 is removed from the valve 100, the valve frame 102 expands towards its deployed state. In one embodiment, the valve frame 102 can be a self-expanding frame that expands once the thread 106 is removed from the frame 102. Alternatively, the valve frame 102 can be a balloon expandable frame, where a balloon is used to expand the frame 102 to its deployed state once the thread 106 has been removed.

In one embodiment, releasing or removing the thread 106 from the valve 100 can be accomplished by sliding the thread 106 over the valve frame 102. For example, one of the first or second ends 120 and 122 of the thread 106 can be pulled to allow the other end of the thread 106 to pass through and away from the valve frame 102. Other ways of removing the thread 106 from the frame 102 are also possible.

As will be appreciated, the thread 106 holding the valve frame 102 can be configured so as not to pinch and/or bind either to itself and/or the frame 102 as the thread 106 is being removed from the frame 102. In one embodiment, this allows the thread 106 to slide over the valve frame 102 as it is removed from the valve frame 102. In an alternative embodiment, the thread 106 can be configured to unravel as it is pulled from the valve frame 102. In one embodiment, the thread 106 can be pulled completely from the valve 100 to allow the valve frame 102 to expand toward the deployed state.

For the various embodiments, the valve frame 102 can be self-expanding and/or balloon expandable. Examples of self-expanding frames include those formed from temperature-sensitive memory alloy that changes shape at a designated temperature or in a temperature range. Alternatively, the self-expanding frames can include those having a spring-bias. Examples of suitable materials include, but are not limited to, medical grade Stainless Steel alloys (304, 316, 17-7 PH, 17-4 PH, etc.), titanium, tantalum, platinum alloys, niobium alloys, Cobalt Alloys (Elgiloy, L605, MP35, etc.), Nb-1Zr, Tungston, Molybdenum, titanium, and combinations thereof. Other polymers, metals and/or metal alloys are also possible. Examples of shape-memory materials include shape memory plastics, polymers, metal alloys, and thermoplastic materials that are inert in the body. Shape memory alloys having superelastic properties generally made from nickel and titanium, commonly known as Nitinol, are also possible materials. Other materials are also possible.

The valve 100 can further include one or more radiopaque markers (e.g., tabs, sleeves, welds). For example, one or more portions of the valve frame 102 can be formed from a radiopaque material. Radiopaque markers can be attached to and/or coated onto one or more locations along the valve frame 102. Examples of radiopaque material include, but are not limited to, gold, tantalum, and platinum. The position of the one or more radiopaque markers can be selected so as to provide information on the position, location and orientation of the valve 100 during its implantation.

The valve 100 further includes the leaflets 104 having surfaces defining a reversibly sealable opening for unidirectional flow of blood through the valve 100. For example, the leaflets 104 can be coupled to the valve frame 102 so as to span and control fluid flow through the lumen 108 of the valve 100. For the present embodiment the valve 100 includes two of the valve leaflets 104 for a bi-leaflet configuration. As appreciated, mono-leaflet, tri-leaflet and/or multileaflet configurations are also possible. Each of the valve leaflets 104 are coupled to the valve frame 102, where the leaflets 104 can repeatedly move between an open state and a closed state for unidirectional flow of blood through a lumen 108 of the valve 100.

In one embodiment, the leaflets 104 can be derived from autologous, allogeneic or xenograft material. As will be appreciated, sources for xenograft material (e.g., valves) include, but are not limited to, mammalian sources such as porcine, equine, and sheep. Additional biologic materials from which to form the valve leaflets 104 include, but are not limited to, explanted veins, pericardium, fascia lata, harvested valves, bladder, vein wall, various collagen types, elastin, intestinal submucosa, and decellularized basement membrane materials, such as small intestine submucosa (SIS), amniotic tissue, or umbilical vein.

Alternatively, the leaflets 104 could be formed from a synthetic material or materials (i.e., composite structures). Possible synthetic materials include, but are not limited to, expanded polytetrafluoroethylene (ePTFE), polytetrafluoroethylene (PTFE), polystyrene-polyisobutylene-polystyrene (SIBS), polyurethane, segmented poly(carbonate-urethane), polyester, polyethlylene (PE), polyethylene terephthalate (PET), silk, urethane, Rayon, Silicone, or the like. In an additional embodiment, the synthetic material can also include metals, such as stainless steel (e.g., 316L) and nitinol. These synthetic materials can be in a woven, a knit, a cast or other known physical fluid-impermeable or permeable configurations. In addition, gold plated metals can be embedded in the leaflet 104 material (e.g., a sandwich configuration) to allow for visualization of the leaflets 104 post placement.

As will be appreciated, the valve 100 can be treated and/or coated with any number of surface or material treatments. Examples of such treatments include, but are not limited to, bioactive agents, including those that modulate thrombosis, those that encourage cellular ingrowth, throughgrowth, and endothelialization, those that resist infection, and those that reduce calcification.

In an additional embodiment, the valve 100 can further include anchors to engage the lumen wall and secure the valve 100 thereto. For example, the valve frame 102 can include barbs that radially extend from the valve 100 to engage the vessel wall in which the valve 100 is implanted.

Figure 2 provides an additional embodiment of a valve 200 of the present disclosure. The valve 200 includes the valve frame 202, valve leaflets 204, and the thread 206 passing over the valve frame 202, as discussed herein. As illustrated, the thread 206 passing over the valve frame 202 has a woven configuration (e.g., a net configuration) over the valve frame 202 that serves to hold the valve frame 202 in a partially deployed state, as discussed herein. In an alternative embodiment, the thread 206 can also weave through the cells 216 of the frame 202 in addition to interweaving with itself. As discussed herein, the weave of the thread 206 can be configured to unravel as the thread 206 is pulled from one of the ends 220 or 222.

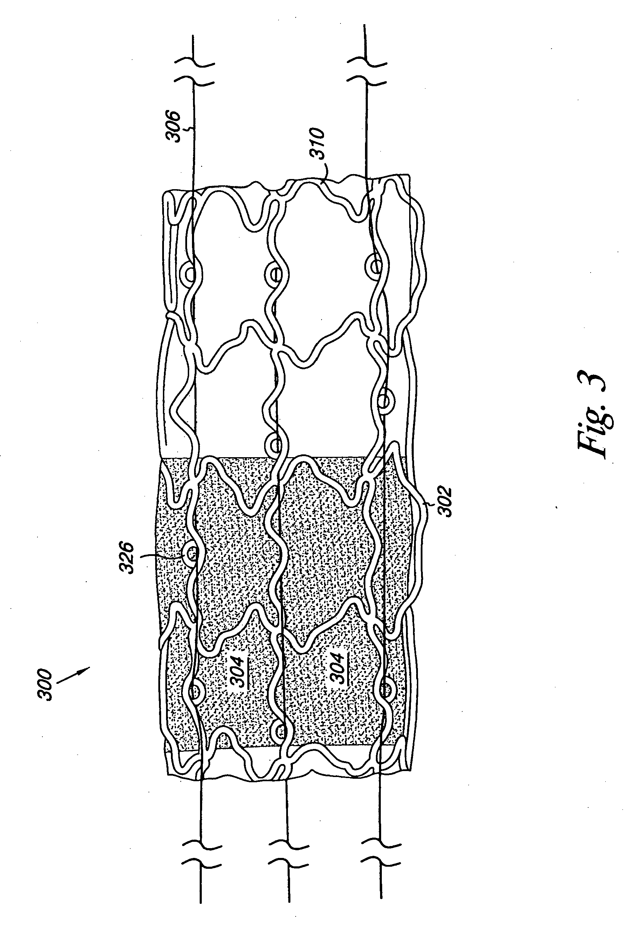

Figure 3 provides another embodiment of a valve 300 of the present disclosure. The valve 300 includes the valve frame 302, valve leaflets 304, and the thread 306 passing over the valve frame 302, as discussed herein. As illustrated, the valve frame 302 includes frame members 310 and eyelets 326. In one embodiment, the eyelet 326 can extend from the frame member 310, as illustrated. Alternatively, the eyelet 326 can be formed in the frame member 310. For example, the eyelet 326 can be an opening through the frame member 310. The thread 306 passes through the valve frame 302 and the eyelets 326 so as to hold the valve frame 302 in the partially deployed state, as discussed herein.

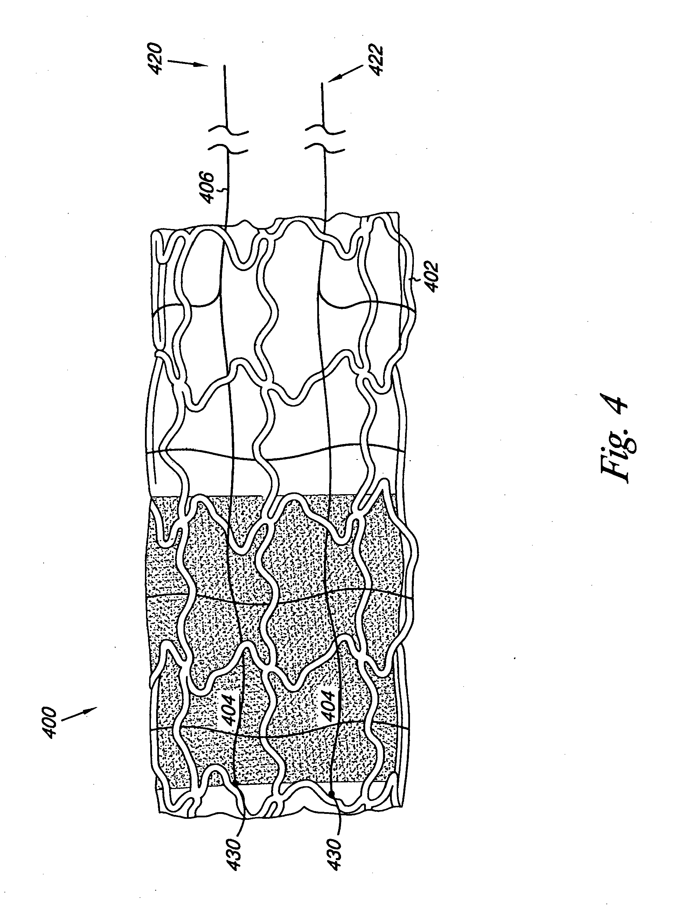

Figure 4 provides an additional embodiment of a valve 400 of the present disclosure. The valve 400 includes the valve frame 402, valve leaflets 404, and the thread 406 passing over the valve frame 402, as discussed herein. As illustrated, the thread 406 passes over the valve frame 402 both in the longitudinal and in the radial direction relative the longitudinal axis of the valve frame 402.

Figure 4 also provides an illustration in which the thread 406 is physically attached to the valve frame at an attachment point 430. In one embodiment, the attachment point 430 serves as an anchor for the thread 406 in holding the valve frame 402 in the partially deployed state. In one embodiment, the thread 406 can be attached to the valve frame 402 at the attachment point 430 having a shape memory polymer (SMP)/alloy collar that was previously pressed into one of the attachment eyelets provided in the stent. The SMP/alloy collar is a temperature sensitive material that upon heating causes the center hole to enlarge and release the thread 406. In one embodiment, heat can be supplied to the SMP/allow collar by delivering a potential through the thread 406 at the attachment point. One or more attachment points 430 can be used to secure the thread 406 to the valve frame 402, as discussed herein.

In one embodiment, heat can be applied to the attachment point 430 by delivering an electrical potential through the thread 406 and across the attachment point 430. For example, the ends 420 and 422 of the thread 406 could be used as poles (e.g., anode and cathode) in delivering the potential across the attachment point 430, where the valve frame 402 serves as a bridge between the connections. The heat generated at the attachment point 430 causes the thread 406 to release from the valve frame 402 so that the thread 406 can be removed from the valve frame 402, as discussed herein. In one embodiment, the thread 406 can have an electrically insulating outer layer to better direct the potential through the attachment points 430.

In an additional embodiment, a Guglielmi electrolytically detachable coil (GDC coil), or mechanism like a GDC coil, can be used at the attachment point 430 for the thread 406. Devices of this nature may be found in U.S. Pat. Nos. 4,994,069; 6,059,779; 5,643,254; 5,423,829; 6,024,754; and 5,522,822 for example. The thread 406 can be released through an electrolytic process in which a small current (1 mA) is provided across a stainless steel wire bridge of the GDC coil. The current causes the wire to dissolve, thereby releasing the thread 406. A positive flush of saline is sent down to site as well so the site does not attract and form thrombus.

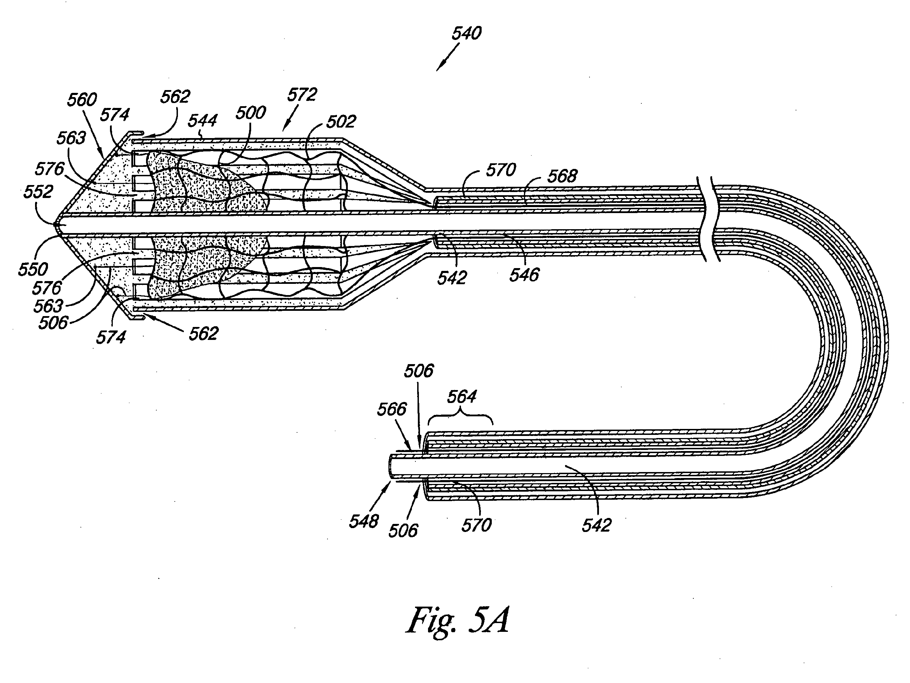

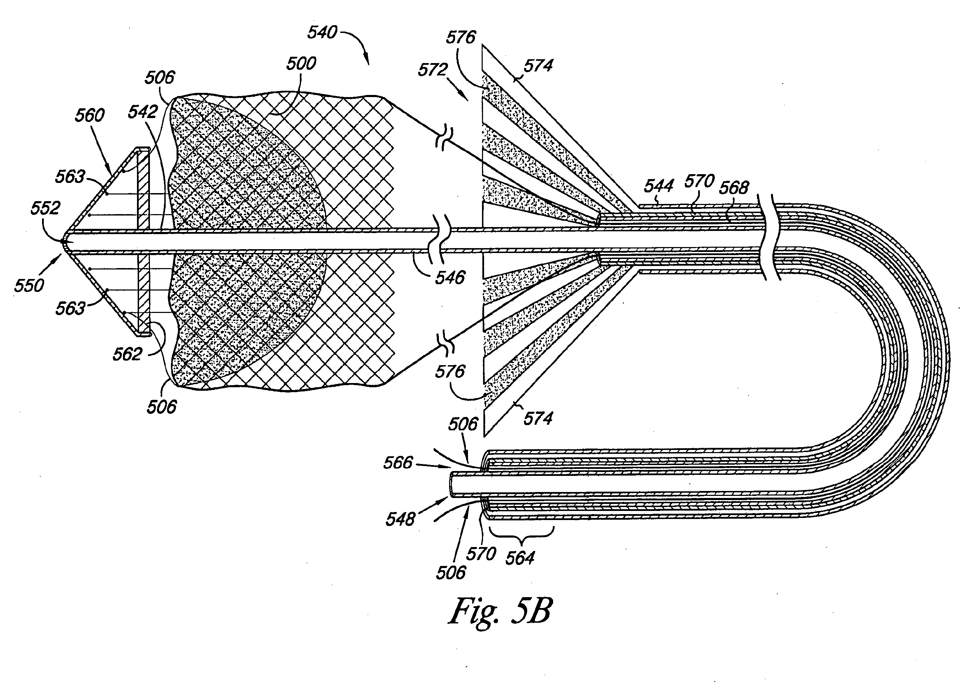

Figures 5A and 5B illustrate a cross-sectional view of an embodiment of a system 540 according to the present disclosure. System 540 includes valve 500, as described herein, releasably joined to an elongate delivery catheter 542. The system also includes a retractable sheath 544, where the valve 500 is releasably positioned between the sheath 544 and the delivery catheter 542. For example, figure 5A illustrates an embodiment in which the retractable sheath 544 is positioned around at least a portion of the delivery catheter 542 to releasably hold the valve 500 in an undeployed state. Figure 5B illustrates an embodiment in which the sheath 544 has been retracted relative the delivery catheter 542 to allow the valve 500 to expand to its partially deployed state.

In the example, the delivery catheter 542 includes an elongate body 546 having a proximal end 548 and a distal end 550. A lumen 552 extends through the proximal and distal ends 548, 550. In one embodiment, the lumen 552 receives a guidewire for guiding the placement of the valve 500 in the vasculature.

For the various embodiments, the elongate delivery catheter 542 also includes a distal tip 560. For the various embodiments, the distal tip 560 has a conical configuration, where the tip 560 has a smaller diameter portion near the distal end 550 of the of the delivery catheter 542 as compared to the proximal portion of the tip 560. The distal tip 560 may also include a recessed lip 562 in which a distal portion of the retractable sheath 544 can releasably seat. In one embodiment, seating the distal portion of the retractable sheath 544 in the recessed lip 562 helps to hold the valve 500 in its undeployed state.

The distal tip 560 also can include anchors 563 through which the thread 506 can pass. In one embodiment, the anchors 563 can be in the form of eyelets through which the thread 506 releasably passes. In an additional embodiment, the anchors 563 can be opening through the distal tip 560 through which the thread 506 releasably passes. Alternatively, the anchors 563 can be one or more of the attachment points and/or structures discussed herein.

The retractable sheath 544 can move longitudinally (e.g., slide) relative the delivery catheter 542 to allow the valve 500 to expand from its undeployed state towards its partially deployed state. In one embodiment, moving the retractable sheath 544 relative the delivery catheter 542 can be accomplished by pulling a proximal portion 564 of the sheath 544 relative a proximal portion 566 of the delivery catheter 542.

As illustrated in figure 5B, the valve 500 expands to its partially deployed state after the retractable sheath 544 has been retracted relative the valve 500. As discussed herein, a thread 506 thread passing over the valve frame 502 and through the anchors 563 restrains the valve 500 in the partially deployed state. As illustrated, the thread 506 extends longitudinally through a lumen 568 of a guide tube 570. The guide tube 570 can extend longitudinally and be concentrically arranged with portions of the delivery catheter 542 and the retractable sheath 544. The thread 506 moves longitudinally within the guide tube 570 to allow the thread 506 to be remove from the valve frame 502 and the anchors 563, as discussed herein.

In an additional embodiment, the guide tube 570 containing the thread 506 can be used to adjust an effective working length of the thread 506. For example, the guide tube 570 can extend through a lumen of the retractable sheath 544 past the proximal portion 564 of the sheath 544. In this configuration, the guide tube 570 can be moved relative the valve 500 to either "shorten" or "lengthen" the effective working length of the thread 506 in holding the valve 500 in the partially deployed state. So, as the tube 570 slides relative to the valve 500 the effective length of the thread 506 allows for adjustments in the percentage of the partially deployed state relative the deployed state.

In an alternative embodiment, the guide tube 570 is in the form of a static collar around the periphery of the delivery catheter 542. For example, the guide tube 570 can extend longitudinally over a small segment of the delivery catheter 542 (e.g., does not extend past the proximal portion 564 of the sheath 544). In this configuration, the guide tube 570 is statically coupled to the delivery catheter 542. Portions of the guide tube 570 also define openings through which the thread 506 travels.

The delivery catheter 542, the retractable sheath 544 and the guide tube 570 can be formed of a number of materials. Materials include polymers, such as PVC, PE, POC, PET, polyamide, mixtures, and block co-polymers thereof. In addition, each of the delivery catheter 542, the retractable sheath 544 and the guide tube 570 can have a wall thickness and an inner diameter sufficient to allow the structures to slide longitudinally relative each other, as described herein, and to maintain both the valve 500 and an expandable filter 572 in compressed states, as discussed herein.

Figures 5A and 5B illustrate the embodiment of the system 540 that includes the expandable filter 572. In one embodiment, the expandable filter 572 forms a portion of the retractable sheath 544. For example, the portion of the retractable sheath 544 forming the filter 572 can include structural members 574 and filter material 576. In one embodiment, the filter material 576 is a flexible material that extends between the structural members 574 to allow fluid flowing through the valve 500 as its being implanted to be filtered.

As illustrated in the embodiment of figure 5A, the structural members 574 include a distal end 576 that is releasably seated in the recessed lip 562 to hold the valve 500 in the undeployed state. When the distal end 576 of the structural members 574 are removed from the lip 562 of the sheath 544, the structural members 574 radiate away from the remainder of the retractable sheath 544 and the elongate delivery catheter 542 to deploy the expandable filter 572, as illustrated in Figure 5B.

Figure 5B also illustrates that the filter 572 is positioned proximal to the valve 500 to allow for larger particles from the fluid flow (e.g., clots and/or debris moving in the blood) to be filtered. In one embodiment, the filter material 576 can have a porosity and/or a mesh size that allows for sufficient fluid flow through the filter 572, while trapping the larger particles from the fluid flow. Such filtering material can be, for example, woven, braided, knit, machined, matted, expanded, or other configurations as are known, or will be known, in polymer and textile processing.

In one embodiment, the structural members 574 of the filter 572 are self-expanding members that can be formed from the structure of the retractable sheath 544. For example, the material forming the retractable sheath 544 can be slit, or cut, to form the structural members 574. The radial pattern of the structural members 574 relative the remainder of the retractable sheath 544 can then be set into the material (e.g., through heat setting).

In an alternative embodiment, the structural members 574 can be formed from temperature-sensitive memory alloy that changes shape at a designated temperature or temperature range. Examples of such materials include, but are not limited to, nitinol and nitinol-type metal alloys. Alternatively, the structural members 574 can include those having a spring-bias imparted into the members forming the filter 572.

As will be appreciated, the filter material 576 can be attached to the structural members 574 in a number of ways. For example, the filter material 576 can be fused to the structural members 574 through the use of an adhesive. Alternatively, the filter material 576 can be fused to the structural members 574 through the use of heat. In an additional embodiment, fasteners can be used to attach the filter material 576 to the structural members 574. Examples of such fasteners include, but are not limited to, staples, stitches, and/or clips.

In addition, the expandable filter 572 in its deployed state (e.g., figure 5B) can apply sufficient pressure to the inner wall of a vascular lumen to reduce the volume of fluid (e.g., blood) that may pass between the filter 572 and the surface of the lumen wall. As will be appreciated, the area and shape defined by the expandable filter 572 (e.g., the diameter of the expandable filter) in its deployed state will be dependent upon the location in which the system is intended to be used.

In one embodiment, the expandable filter 572 can be removed from the body by advancing an additional retraction sheath over the retractable sheath 544 to collapse and house the expandable filter 572 between the retraction sheath and the sheath 544. In an additional embodiment, the distal portion of the retractable sheath 544 can also be configured as a temporary valve. Embodiments of such a valve structure can be found in co-pending US Patent Application No. 11/049,019 entitled "Filter System and Method".

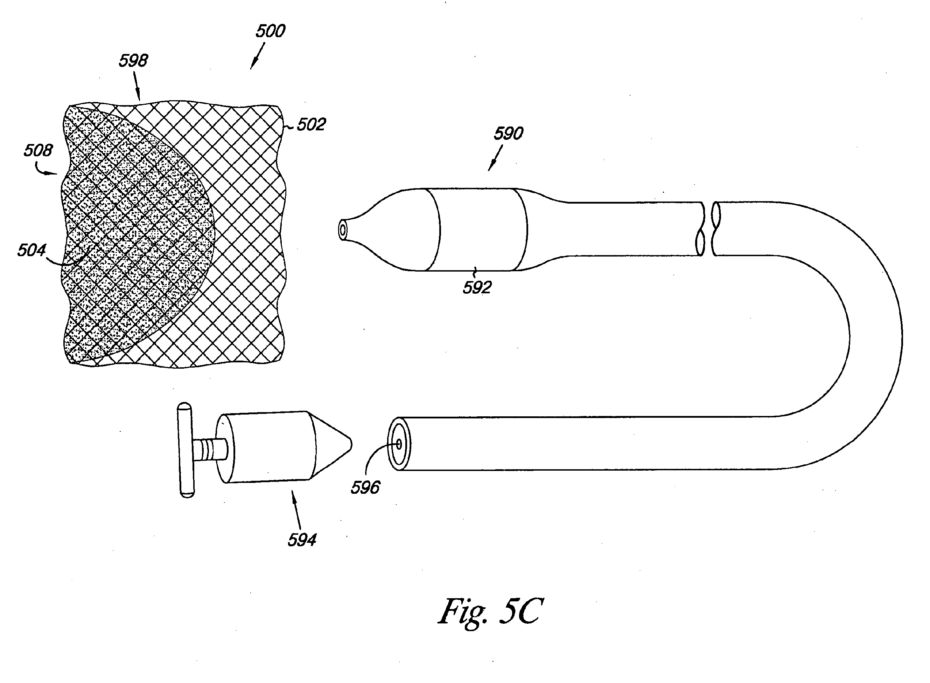

As discussed herein, removing the thread 506 from the valve frame 502 allows the valve 500 to expand towards its deployed state. In an additional embodiment, seating of the valve 500 in its deployed state within the vasculature can be assisted by radially expanding the valve 500 with a balloon catheter. For example, figure 5C provides an illustration of the valve 500 after the thread 506 has been removed from the valve frame 502. A balloon catheter 590 having an inflatable balloon 592 can be positioned in the lumen 508 of the valve 500. The balloon 592 can be inflated with fluid supplied by an inflation device 594 through catheter lumen 596 in fluid communication with the balloon 592. The balloon 592 can then contact and radially expand the valve frame 502 to better ensure that the valve 500 is deployed.

In an additional embodiment, the valve 500 can further include a sealing material 598 positioned on the periphery of the valve frame 502. In one embodiment, once implanted against the tissue the sealing material 598 can swell due to the presence of blood to occupy volume between the valve frame 502 and the tissue on which the valve 500 has been implanted so as to prevent leakage of the blood around the outside of the valve 500. In one embodiment, the sealing material can be attached to the frame 502 where the valve leaflets 504 are attached to the valve frame 502.

A variety of suitable materials for the sealing material 598 are possible. For example, the sealing material 598 can be selected from the general class of materials that include polysaccharides, proteins, and biocompatible gels. Specific examples of these polymeric materials can include, but are not limited to, those derived from poly(ethylene oxide) (PEO), PET, poly(ethylene glycol) (PEG), poly(vinyl alcohol) (PVA), poly(vinylpyrrolidone) (PVP), poly(ethyloxazoline) (PEOX) polyaminoacids, pseudopolyamino acids, and polyethyloxazoline, as well as copolymers of these with each other or other water soluble polymers or water insoluble polymers. Examples of the polysaccharide include those derived from alginate, hyaluronic acid, chondroitin sulfate, dextran, dextran sulfate, heparin, heparin sulfate, heparan sulfate, chitosan, gellan gum, xanthan gum, guar gum, water-soluble cellulose derivatives, and carrageenan. Examples of proteins include those derived from gelatin, collagen, elastin, zein, and albumin, whether produced from natural or recombinant sources.

The embodiments of the valve described herein may be used to replace, supplement, or augment valve structures within one or more lumens of the body. For example, embodiments of the present invention may be used to replace an incompetent valve of the heart, such as the aortic, pulmonary and/or mitral valves of the heart. In one embodiment, the native valve can remain unaltered or be altered through a valvoplasty procedure prior to implanting the valve of the present disclosure.

In addition, positioning the system having the valve as discussed herein includes introducing the system into the cardiovascular system of the patient using minimally invasive percutaneous, transluminal techniques. For example, a guidewire can be positioned within the cardiovascular system of a patient that includes the predetermined location. The system of the present disclosure, including the valve as described herein, can be positioned over the guidewire and the system advanced so as to position the valve at or adjacent the predetermined location. In one embodiment, radiopaque markers on the catheter and/or the valve, as described herein, can be used to help locate and position the valve.

The valve can be deployed from the system at the predetermined location in any number of ways, as described herein. In one embodiment, the valve of the present disclosure can be deployed and placed in any number of cardiovascular locations. For example, valve can be deployed and placed within a major artery of a patient. In one embodiment, major arteries include, but are not limited to, the aorta. In addition, valves of the present invention can be deployed and placed within other major arteries of the heart and/or within the heart itself, such as in the pulmonary artery for replacement and/or augmentation of the pulmonary valve and between the left atrium and the left ventricle for replacement and/or augmentation of the mitral valve. Other locations are also possible.

As discussed herein, the valve can be deployed in a staged fashion. In the first stage, the valve is held in its undeployed state (e.g., compressed state) by the retractable sheath. The retractable sheath can then be moved (e.g., retracting the sheath) to allow the valve to radially expand from the undeployed state. In one embodiment, this can be done through the use of a self-expanding valve frame. Alternatively, a balloon catheter could be used to expand the valve frame to the partially deployed state. The thread(s) then restrain or hold the radial expansion at the partially deployed state. Upon removal of the thread(s), the valve then expands toward its deployed state, either through self-expansion and/or through balloon expansion, as discussed herein. In an additional embodiment, the valve can also be radially expanded with an inflatable balloon to set the valve in the deployed state.

Once implanted, the valve can provide sufficient contact with the body lumen wall to reduce the volume of retrograde flow around the valve and the body lumen wall, and to securely locate the valve and prevent migration of the valve. It is, however, understood that some leaking or fluid flow may occur between the valve and the body lumen and/or through valve leaflets. In one embodiment, the valve frame can also include anchors (e.g., barbs) that extend radially from the frame to engage the lumen wall and secure the valve thereto. The valve described herein also displays sufficient flexibility and resilience so as to accommodate changes in the body lumen diameter, while maintaining the proper placement of valve.

It is understood that the threads of the present disclosure could be used to retract and/or retrieve the valve from the partially deployed state to a condition in which the valve could be removed from the patient.

## Claims

1. A system (540), comprising:
an elongate delivery catheter (542);
a retractable sheath (544) positioned around at least a portion of the elongate delivery catheter (542), where the retractable sheath (544) moves longitudinally relative the elongate delivery catheter (542);
a valve (100, 200, 300, 400, 500) positioned between the elongate delivery catheter (542) and the retractable sheath (544), where the valve (100, 200, 300, 400, 500) includes a valve frame (102,202, 302, 402, 502) and a valve leaflet (104, 204, 304, 404, 504) coupled to the valve frame (102, 202, 302, 402, 502), and where the valve frame has a first end and second end with a longitudinal length between the first end and the second end;
and **characterised in that** it also comprises a thread (106, 206, 306, 406, 506) extending between the first end and the second end of the valve frame (102, 202, 302, 402, 502) to restrain the valve (100) in a partially deployed state, wherein the thread is adapted to be removed to allows the valve frame to expand towards a deployed state, and wherein the valve (100) is adapted to expands from an undeployed state when positioned between the elongate delivery catheter (542) and the retractable sheath (544) to the partially deployed state when the retractable sheath (544) slides longitudinally relative the elongate delivery catheter (542) to release the valve (100).

2. The system (540) of claim 1, where the retractable sheath (544) includes an expandable filter (572), where the expandable filter (572) includes filter material (576) extending between structure members (574) that radiates away from the elongate delivery catheter (542) to deploy the expandable filter (572).

3. The system (540) of any one of the preceding claims, where the elongate delivery catheter (542) includes a distal tip (560) having a recessed lip (562), where a distal end (576) of the structural members (574) of the expandable filter (572) releasably seat in the recessed lip (562) to hold the valve (100) in the undeployed state.

4. The system (540) of any one of the preceding claims, including a balloon catheter (590) having an inflatable balloon (592), where the inflatable balloon (592) is positioned inside a lumen (508) of the valve frame (102, 202, 302, 402, 502), where the inflatable balloon (592) is adapted to expand to fully deploy the valve (100).

5. The system (540) of any one of the preceding claims, where the retractable sheath (544) can move longitudinally relative the elongate delivery catheter (542).

6. The system (540) of any one of the preceding claims, where the valve (100, 200, 300, 400, 500) is held in a compressed state between the elongate delivery catheter (542) and the retractable sheath (544).

7. The system (540) of claim 6, where the thread restrains the valve frame (102, 202, 302, 402, 502) in a state having a radial diameter that is greater than the radial diameter of the valve frame (102, 202, 302, 402, 502) in the compressed state.

8. The system (540) of any one of the preceding claims, where the valve frame (102, 202, 302, 402, 502) includes frame members (110, 310) defining cells, where the thread (106, 206, 306, 406, 506) passing over the valve frame (102, 202, 302, 402, 502) passes through the cells.

9. The system (540) of any one of the preceding claims, where the frame members (110, 310) include an inner surface (112) and an outer surface (114), where the thread (106, 206, 306, 406, 506) passes over a portion of the inner surface (112) and a portion of the outer surface (114) of the frame member (110, 310).

10. The system (540) of any one of the preceding claims, where the thread (106, 206, 306, 506) passing over the valve frame (102, 202, 302, 402, 502) has a woven configuration.

11. The system (540) of claim 10, where the woven configuration forms a net in which the valve frame (102) is held in the partially deployed state.

12. The system (540) of any one of the preceding claims, where the valve frame (102, 202, 302, 402, 502) includes eyelets (326) through which the thread (106, 206, 306, 406, 506) passes to hold the valve frame (102) in the partially deployed state.

13. The system (540) of any one of the preceding claims, where the valve (100, 200, 300, 400, 500) is held by the thread in a state that is larger than when positioned between sheath and catheter.

14. The system (540) of any one of the preceding claims, where the valve frame (102, 202, 302, 402, 502) holds the thread under tension in the partially deployed state.

15. The system (540) of any one of the preceding claims, where the thread extends longitudinally through a lumen of a guide tube (570).

## Patentansprüche

1. System (540), das aufweist:
einen länglichen Abgabekatheter (542);
eine zurückziehbare Hülle (544), die um mindestens einen Abschnitt des länglichen Abgabekatheters (542) positioniert ist, wobei sich die zurückziehbare Hülle (544) relativ zum länglichen Abgabekatheter (542) längs bewegt;
eine Klappe (100, 200, 300, 400, 500), die zwischen dem länglichen Abgabekatheter (542) und der zurückziehbaren Hülle (544) positioniert ist, wobei die Klappe (100, 200, 300, 400, 500) einen Klappenrahmen (102, 202, 302, 402, 502) und ein mit dem Klappenrahmen (102, 202, 302, 402, 502) gekoppeltes Klappensegel (104, 204, 304, 404, 504) aufweist und wobei der Klappenrahmen ein erstes Ende und zweites Ende mit einer Länge in Längsrichtung zwischen dem ersten Ende und dem zweiten Ende hat;
und **dadurch gekennzeichnet, dass** es ferner einen Faden (106, 206, 306, 406, 506) aufweist, der sich zwischen dem ersten Ende und dem zweiten Ende des Klappenrahmens (102, 202, 302, 402, 502) erstreckt, um die Klappe (100) in einem teilweise entfalteten Zustand zurückzuhalten, wobei der Faden geeignet ist, entfernt zu werden, damit der Klappenrahmen in einen entfalteten Zustand expandieren kann, und wobei die Klappe (100) geeignet ist, aus einem nicht entfalteten Zustand bei Positionierung zwischen dem länglichen Abgabekatheter (542) und der zurückziehbaren Hülle (544) in den teilweise entfalteten Zustand zu expandieren, wenn die zurückziehbare Hülle (544) relativ zum länglichen Abgabekatheter (542) längs gleitet, um die Klappe (100) freizugeben.

2. System (540) nach Anspruch 1, wobei die zurückziehbare Hülle (544) ein expandierbares Filter (572) aufweist, wobei das expandierbare Filter (572) Filtermaterial (576) aufweist, das sich zwischen Strukturbauteilen (574) erstreckt, die vom länglichen Abgabekatheter (542) weg strahlenförmig ausgehen, um das expandierbare Filter (572) zu entfalten.

3. System (540) nach einem der vorstehenden Ansprüche, wobei der längliche Abgabekatheter (542) eine distale Spitze (560) mit einer eingelassenen Lippe (562) aufweist, wobei ein distales Ende (576) der Strukturbauteile (574) des expandierbaren Filters (572) in der eingelassenen Lippe (562) lösbar sitzt, um die Klappe (100) im nicht entfalteten Zustand zu halten.

4. System (540) nach einem der vorstehenden Ansprüche, das einen Ballonkatheter (590) mit einem inflatierbaren Ballon (592) aufweist, wobei der inflatierbare Ballon (592) innerhalb eines Lumens (508) des Klappenrahmens (102, 202, 302, 402, 502) positioniert ist, wobei der inflatierbare Ballon (592) geeignet ist zu expandieren, um die Klappe (100) vollständig zu entfalten.

5. System (540) nach einem der vorstehenden Ansprüche, wobei sich die zurückziehbare Hülle (544) relativ zum länglichen Abgabekatheter (542) längs bewegen kann.

6. System (540) nach einem der vorstehenden Ansprüche, wobei die Klappe (100, 200, 300, 400, 500) zwischen dem länglichen Abgabekatheter (542) und der einziehbaren Hülle (544) in einem komprimierten Zustand gehalten wird.

7. System (540) nach Anspruch 6, wobei der Faden den Klappenrahmen (102, 202, 302, 402, 502) in einem Zustand zurückhält, der einen Radialdurchmesser hat, der größer als der Radialdurchmesser des Klappenrahmens (102, 202, 302, 402, 502) im komprimierten Zustand ist.

8. System (540) nach einem der vorstehenden Ansprüche, wobei der Klappenrahmen (102, 202, 302, 402, 502) Zellen definierende Rahmenbauteile (110, 310) aufweist, wobei der über dem Klappenrahmen (102, 202, 302, 402, 502) hinweglaufende Faden (106, 206, 306, 406, 506) die Zellen durchläuft.

9. System (540) nach einem der vorstehenden Ansprüche, wobei die Rahmenbauteile (110, 310) eine Innenfläche (112) und eine Außenfläche (114) aufweisen, wobei der Faden (106, 206, 306, 406, 506) über einem Abschnitt der Innenfläche (112) und einem Abschnitt der Außenfläche (114) des Rahmenbauteils (110, 310) hinwegläuft.

10. System (540) nach einem der vorstehenden Ansprüche, wobei der über dem Klappenrahmen (102, 202, 302, 402, 502) hinweglaufende Faden (106, 206, 306, 406, 506) eine Webkonfiguration hat.

11. System (540) nach Anspruch 10, wobei die Webkonfiguration ein Netz bildet, in dem der Klappenrahmen (102) im teilweise entfalteten Zustand gehalten wird.

12. System (540) nach einem der vorstehenden Ansprüche, wobei der Klappenrahmen (102, 202, 302, 402, 502) Ösen (326) aufweist, die der Faden (106, 206, 306, 406, 506) durchläuft, um den Klappenrahmen (102) im teilweise entfalteten Zustand zu halten.

13. System (540) nach einem der vorstehenden Ansprüche, wobei die Klappe (100, 200, 300, 400, 500) durch den Faden in einem Zustand gehalten wird, der größer als bei Positionierung zwischen Hülle und Katheter ist.

14. System (540) nach einem der vorstehenden Ansprüche, wobei der Klappenrahmen (102, 202, 302, 402, 502) den Faden in teilweise entfalteten Zustand unter Zugspannung hält.

15. System (540) nach einem der vorstehenden Ansprüche, wobei sich der Faden durch ein Lumen eines Führungsschlauchs (570) längs erstreckt.

## Revendications

1. Système (540), comprenant :
un cathéter d'administration oblong (542) ;
une gaine rétractable (544) disposée autour d'au moins une partie du cathéter d'administration oblong (542), la gaine rétractable (544) étant mobile longitudinalement par rapport au cathéter d'administration oblong (542) ;
une valve (100, 200, 300, 400, 500) disposée entre le cathéter d'administration oblong (542) et la gaine rétractable (544), ladite valve (100, 200, 300, 400, 500) comportant un cadre de valve (102, 202, 302, 402, 502) et un feuillet de valve (104, 204, 304, 404, 504) accouplé au cadre de valve (102, 202, 302, 402, 502), le cadre de valve ayant une première et une deuxième extrémités avec une longueur longitudinale s'étendant entre la première et la deuxième extrémités ; et
**caractérisé en ce qu'**il comprend également un fil (106, 206, 306, 406, 506) s'étendant entre la première et la deuxième extrémités du cadre de valve (102, 202, 302, 402, 502) pour maintenir la valve (100) dans un étant partiellement déployé, le fil étant prévu pour être retiré afin de permettre une expansion du cadre de valve jusqu'à un état de déploiement, et la valve (100) étant prévue pour être expansée d'un état non déployé quand elle est positionnée entre le cathéter d'administration oblong (542) et la gaine rétractable (544) vers l'état partiellement déployé quand la gaine rétractable (544) coulisse longitudinalement par rapport au cathéter d'administration oblong (542) pour libérer la valve (100).

2. Système (540) selon la revendication 1, où la gaine rétractable (544) comporte un filtre expansible (572), ledit filtre expansible (572) comportant un matériau de filtre (576) s'étendant entre des éléments structurels (574) disposés en rayons à partir du cathéter d'administration oblong (542) afin de déployer le filtre expansible (572).

3. Système (540) selon l'une des revendications précédentes, où le cathéter d'administration oblong (542) comporte un embout distal (560) avec un rebord en retrait (562), une extrémité distale (576) des éléments structurels (574) du filtre expansible (572) étant logée de manière amovible dans le rebord en retrait (562) pour maintenir la valve (100) en état non déployé.

4. Système (540) selon l'une des revendications précédentes, comportant un cathéter à ballonnet (590) avec un ballonnet gonflable (592), ledit ballonnet gonflable (592) étant disposé à l'intérieur d'une lumière (508) du cadre de valve (102, 202, 302, 402, 502), ledit ballonnet gonflable (592) étant prévu pour être expansé pour un déploiement complet de la valve (100).

5. Système (540) selon l'une des revendications précédentes, où la gaine rétractable (544) est déplaçable longitudinalement par rapport au cathéter d'administration oblong (542).

6. Système (540) selon l'une des revendications précédentes, où la valve (100, 200, 300, 400, 500) est maintenue dans un état comprimé entre le cathéter d'administration oblong (542) et la gaine rétractable (544).

7. Système (540) selon la revendication 6, où le fil maintient le cadre de valve (102, 202, 302, 402, 502) dans un état où un diamètre radial est supérieur au diamètre radial du cadre de valve (102, 202, 302, 402, 502) en état comprimé.

8. Système (540) selon l'une des revendications précédentes, où le cadre de valve (102, 202, 302, 402, 502) comporte des éléments de cadre (110, 310) définissant des cellules, lesdites cellules étant traversées par le fil (106, 206, 306, 406, 506) s'étendant au-dessus du cadre de valve (102, 202, 302, 402, 502).

9. Système (540) selon l'une des revendications précédentes, où les éléments de cadre (110, 310) comportent une surface intérieure (112) et une surface extérieure (114), le fil (106, 206, 306, 406, 506) s'étendant au-dessus d'une partie de la surface intérieure (112) et d'une partie de la surface extérieure (114) de l'élément de cadre (110, 310).

10. Système (540) selon l'une des revendications précédentes, où le fil (106, 206, 306, 406, 506) s'étendant au-dessus du cadre de valve (102, 202, 302, 402, 502) a une structure tissée.

11. Système (540) selon la revendication 10, où la structure tissée forme un réseau où le cadre de valve (102) est maintenu en état partiellement déployé.

12. Système (540) selon l'une des revendications précédentes, où le cadre de valve (102, 202, 302, 402, 502) présente des oeillets (326) traversés par le fil (106, 206, 306, 406, 506) pour maintenir le cadre de valve (102) en état partiellement déployé.

13. Système (540) selon l'une des revendications précédentes, où la valve (100, 200, 300, 400, 500) est maintenue par le fil dans un état où elle est plus grande que quand elle est positionnée entre la gaine et le cathéter.

14. Système (540) selon l'une des revendications précédentes, où le cadre de valve (102, 202, 302, 402, 502) maintient le fil sous tension en état partiellement déployé.

15. Système (540) selon l'une des revendications précédentes, où le fil s'étend longitudinalement dans une lumière d'un tube de guidage (570).
